Europäisches Patentamt

(19) European Patent Office

Office européen des brevets

(11) Publication number: **0 021 828**

**A2**

(12) # EUROPEAN PATENT APPLICATION

(21) Application number: 80302126.0

(22) Date of filing: 25.06.80

(51) Int. Cl.³: **A 61 L 2/04**
**G 02 C 13/00**

(30) Priority: 25.06.79 US 51765

(43) Date of publication of application:
07.01.81 Bulletin 81/1

(84) Designated Contracting States:
DE FR GB IT

(71) Applicant: ACDC STERILENS LIMITED
450 Midtown Centre
Regina Saskatchewan(CA)

(72) Inventor: Ashley, Ward Richard
1621 West 13th Street
Wilmington, Delaware 19806(US)

(72) Inventor: Hladky, Joseph
2265 Harvey Street
Regina, Saskatchewan(CA)

(74) Representative: Fisher, Bernhard et al,
B. FISHER & CO. 36 Sydenham Road
Croydon Surrey CR0 2EF(GB)

(54) Contact lens sterilizing unit.

(57) A contact lens heat disinfecting unit having features which allow contact lenses to be disinfected effectively by an electrical heating arrangement which is designed to use minimum energy so that the unit can be operated on its own self-contained batteries. The unit includes a casing having recesses for locating protuding portions of a contact lens holder, with an electrical resistance heater mounted within each of the recesses so as to contact the protuding portions of the lens holder when located in position. The heater is a flexible element mounted on a resilient heat insulating base in such manner as to be thermally isolated from any substantial metal body and such as to be held in contact with the surface of the lens holder when the latter is in place. The holder itself is designed to have a minimum heat capacity, and such that the lens held within the holder is positioned close to those parts of the holder in contact with the heater.

FIG. I

- 1 -

Contact Lens Sterilizing Unit

The present invention relates to apparatus for hot disinfection of soft hydrophilic contact lenses.

Soft hydrophilic contact lenses must be frequently disinfected, preferably daily. Disinfection can be carried out by a cold system using a saline solution preserved with strong chemicals to ensure long shelf life as well as high bacteriocidal effect. Although this system allows portability, there are disadvantages arising from the cost of replacing the solution and reaction of wearers to the chemicals which tend to be absorbed by the lens. The use of these chemicals is avoided by the hot disinfection method wherein the lenses are disinfected by being heated within a liquid. Examples of prior patents showing apparatus for heat disinfection or sterilization of contact lenses are the following U.S. patents:-

      Patent 3,983,362 which issued September 28, 1976
          to Bausch & Lomb, Inc.;

      Patent 3,998,590 issued December 21, 1976 to
          Glorieux;

      Patent 4,044,226 which issued August 23, 1977 to
          Bausch & Lomb, Inc.; and

      Patent 4,080,167 issued March 21, 1978 to Beers All of the devices shown in these patents, and in fact all devices for heat disinfection of contact lenses which are known to the applicants, require a mains supply of electricity. The present invention provides a disinfecting unit which is not restricted to the use of main supply electricity, but which may be operated on its own self-contained battery, which is preferably rechargeable. This is achieved by a combination of special features which allow disinfection to be effected with minimum use of electrical energy. The apparatus also has an additional advantage in having a short working cycle as compared to presently available apparatus.

In accordance with one broad aspect of the present invention there is provided apparatus for disinfecting contact lenses including in combination a lens holder having a protuding hollow receptacle and means for locating a contact lens close to the inner surface of this receptacle, and a heat disinfecting unit having a casing with a recess shaped to receive the outer surface of the protuding hollow receptacle, this recess including an electrical heater arranged to contact a substantial part (preferably at least one half and in the preferred embodiment about two-thirds) of the outer surface of the receptacle for transmitting heat thereto, thus disinfecting the lens held by the holder. The heater includes an electrical conductor thermally isolated from any substantial metal body and separated by thermal insulation from the casing.

The electrical heater is a thin, flexible element mounted on a resilient thermally insulating base, the flexibility of the heating element in relation to the resilience of the insulating base being such that the heater tends to

- 3 -

conform to the shape of the adjacent surface of the lens
holder when the latter is held in place in the recess.
The base has a thickness considerably greater than that
of the heater and is such that pressure applied to discrete areas of the heater cause this to be deformed
from the relaxed shape by an amount at least equivalent
to its thickness. Means are provided for retaining the
lens holder in pressure engagement with the heater.

The lens holder normally has two protuding receptacles
one  for each of two lenses, and the unit has two recesses
one for each receptacle, which may be arranged within a
larger recess.

The electrical heater is preferably a flat heating element of metal foil preferably of less than 0.002 inches
(0.051 mm) covered with a layer of insulating material
such as synthetic rubber. Such a heater may
be quite thin, preferably less than 0.05 inches (1.25 mm)
in thickness, so as to have a minimum heat capacity. The
resilient insulating base is preferably formed of sponge
rubber such as to press the heater into firm contact
with the surface of the lens holder and to effectively
insulate the heater from the remainder of the unit so
that there is little heat loss to any part of the unit.
The lens holder itself, which is also a novel unit,
includes a plate having two hollow receptacles protuding
from the underside thereof, the upper ends of the receptacles protuding above the plate and having removable
screw caps each cap carrying a lens support of convex
form, the lens support occupying a large part of the total
internal volume of the receptacle, so that the heat
capacity of this liquid is also minimized. The lens
support may for example occupy about one third the volume
of the receptacle.

- 4 -

The main features of the invention described broadly above allow contact lenses held by the holder to be disinfected with minimum wastage of heat. The direct contact between the heater and the lens holder, and the closeness of the lens to the inner wall of the lens holder, ensures that heat transfer is good, and in addition the heat capacity of the heater and of the lens holder is minimized. By this means, a unit is provided which can operate through a useful number of cycles, say at least 10 cycles by energy supplied from batteries held by the unit and without requiring recharging of such batteries. The arrangement in accordance with this invention also allows disinfecting to be completed in a relatively short time, say about 10 to 15 minutes.

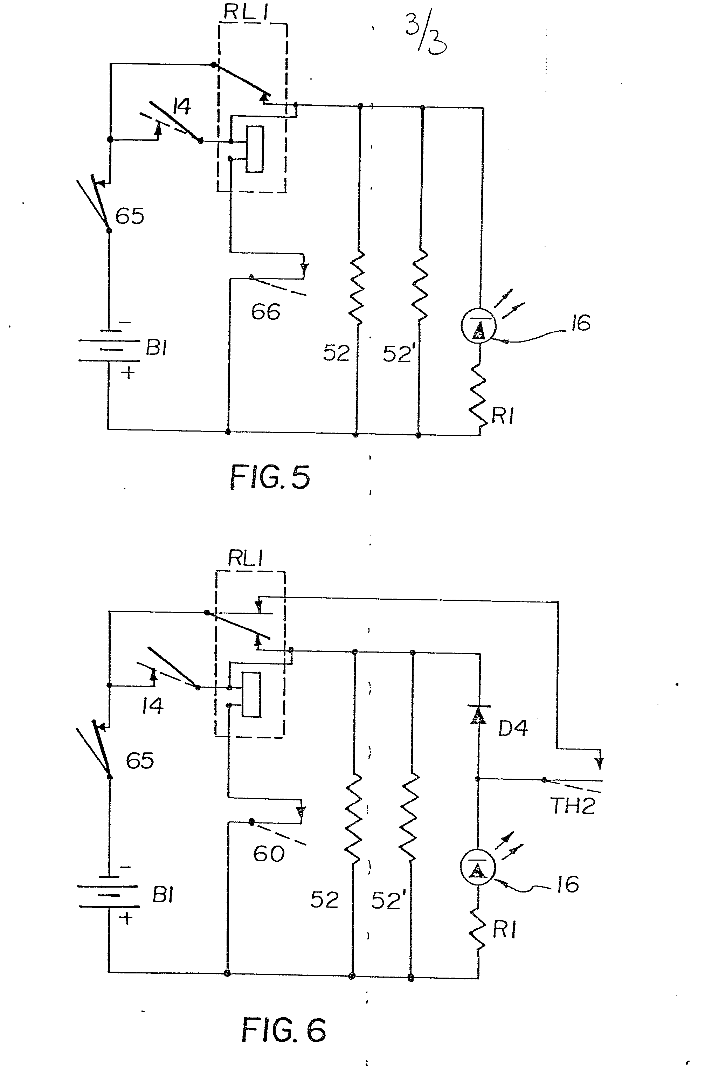

The apparatus of this invention may instructively be compared for example with the commercially available unit which is described in U.S. patent 4,044, 226 referred to above. In this, a contact lens holder is placed within a recess of a heat member 16 which is deliberately chosen so as to have a fairly high heat capacity. Since this member is of solid metal the contact which is produced between this member and a lens holder will not be a complete surface to surface contact. A unit of this kind would be quite unsuitable for operating with self-contained batteries, since the amount of energy required to heat up the heat member 16 would be excessive. As may be seen from Column 6 of the patent, in reference to Fig. 5, electrical energy will normally be supplied to the heat member 16 for about 15 minutes to achieve the disinfecting, and after this the apparatus has to be allowed to cool for perhaps 30 minutes before the lens holder is cool enough to be handled safely. It may be understood from this comparison that not only is the apparatus of this invention unique in its suitability for use with batteries, but will also operate on a much shorter time cycle.

Although other prior art patents, for example U.S. patents 3,998,590 and 4,080,167 referred to above, do not use a large heat capacity metal member, nevertheless they do use a relatively large volume of liquid which also requires to be heated.

The present invention will be described in further detail with reference to the accompanying drawings in which:-

Fig. 1 shows a perspective view of the heat disinfecting unit with a lens holder shown removed from the unit;

Fig. 2 shows a view, partly disassembled, of a lens holder suitable for use with this unit;

Fig. 3 shows a sectional view through the heat disinfecting unit as on lines 3-3 of Fig. 1 but with a lens holder in place;

Fig. 4 shows an enlarged detail sectional view of the heater unit and its mounting;

Fig. 5 shows an electrical circuit for the heat disinfecting unit; and

Fig. 6 shows an alternative circuit for the unit.

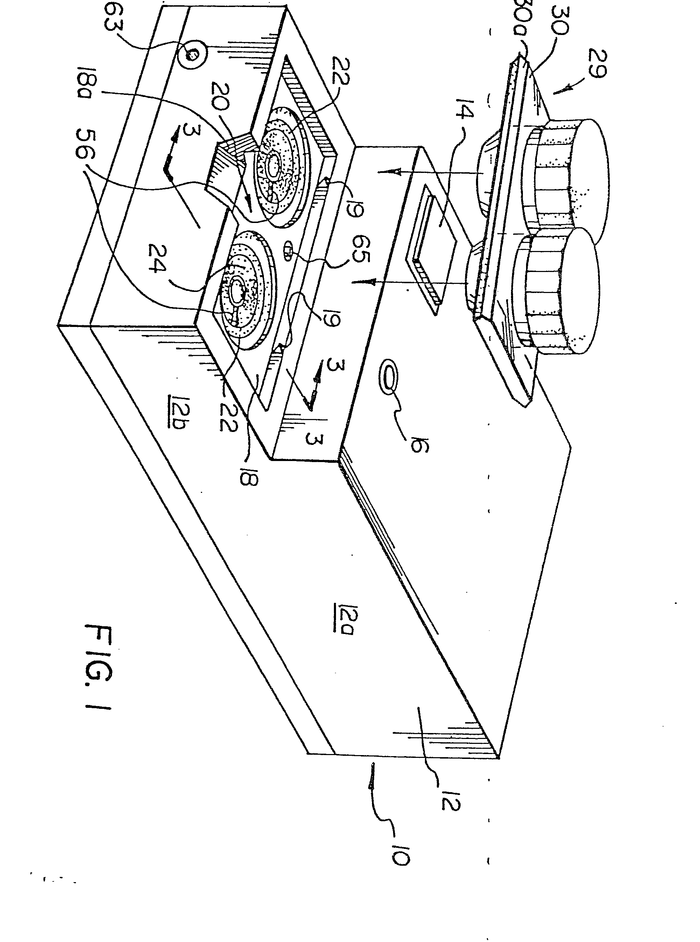

As shown in Fig. 1, the heat disinfecting unit 10 has a plastic casing 12 of generally rectangular form and having a main body portion 12a and a forward body portion 12b which is of lesser height than 12a. The upper front part of portion 12a carries a push button 14, and an indicating light in the form of a light emitting diode 16.

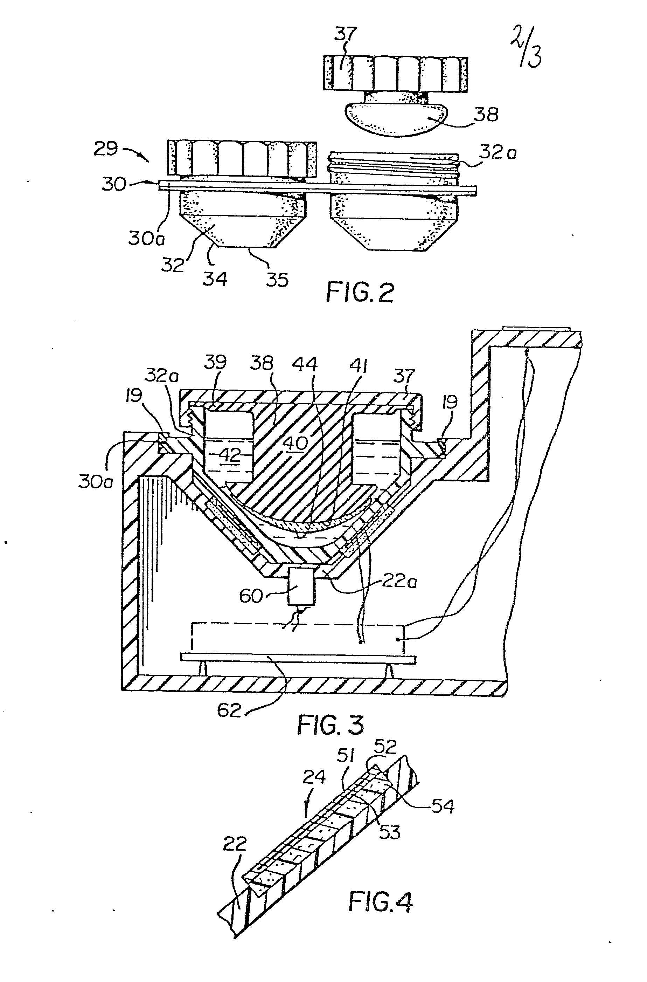

The upper surface of portion 12b has a rectangular recess 18 for receiving a lens holder indicated at 29. The front and rearward edges of this recess are provided with slanting lugs 19, shown more clearly in Fig. 3, for retaining the lens holder. The front of the recess communicates with a finger well 18a to allow removal of the lens holder. Within the rectangular recess is a flat surface 20 interrupted by two circular, frusto-conical recesses 22. The conical surfaces of these recesses each contain a heater 24, which will be described in more detail with reference to Figs. 3 and 4.

Turning now to the lens holder for an understanding of how this cooperates with the recesses 22, this holder is shown in Fig. 2. The main body of the holder is formed of plastics and includes a rectangular flat plate 30 with which are integrally moulded two hollow receptacles 32 protuding downwardly from the plate. Each of these receptacles has a frusto-conical wall 34 which closely matches the dimensions of the internal surfaces of the recesses 22, and each receptacle has a flat bottom 35 allowing the holder to rest on a flat surface. Protuding upwardly from plate 30 are two cylindrical extensions 32a of the hollow receptacles which have an external screw thread engageable by a screw cap 37. As best seen in Fig. 3, each screw cap carries a lens support in the form of a rubber element 38 having a disc-like top portion 39 which is an interference fit within the screw cap, and depending from this portion 39 is a cylindrical extension 40 leading to a convex lens supporting surface 41. As will be seen in Fig. 3 a contact lens 44 placed on this surface 41 normally remains in place although it is readily removable. The outer edges of the disc portion 39 provide a resilient

- 7 -

sealing gasket between the cap and the holder portion
32a so that liquid 42 (for example a saline solution)
which substantially fills the holder is prevented
from leaking out.

It should be noted firstly that the lens supports 40 are
designed to hold the contact lenses close to the conical
walls 34 of the receptacles so that the outer surface of
a contact lens is spaced by no more than 0.1 inch (2.5 mm)
from the inner surface of its receptacle. Secondly, it
will be seen that a relatively large proportion (say at
least 30%) of the internal volume of the receptacle is
occupied by this support 40 so that the amount of liquid
required to fill the receptacle is minimized.

As also seen in Fig. 3, each of the longer edges of
the holder plate 30 are provided with a sloped surface
30a for engaging as a snap-in fit with the lugs 19 of
the rectangular recess in the heating unit. There is
enough resilience in the parts to allow the holder to be
removed by a finger placed within the well. The lugs
19 operate to hold the conical walls 34 of the receptacles in pressure engagement with the heaters 24.

As mentioned, the heaters 24 are exposed within the
conical surfaces of the recesses, and the nature of and
mounting of the heaters is best shown in Fig. 4. As
shown, the casing walls surrounding the recesses are
each provided with an annular depression which retains
a unit including heater 24 and insulating pad. This
unit is made up of the following layers of material,
starting from the top:-
    A sheet of silicone rubber 51 of 0.015 inches
        (0.38 mm) thickness
    A heating element 52 in the form of a metal flat foil
        of 0.001 inch (0.025 mm) thickness

- 8 -

A layer of silicone rubber and silicone rubber
adhesive 53 of 0.020 inches (0.51 mm) thickness
A silicone rubber sponge pad 54 of 0.062 inches
(1.55 mm) thickness, and
A silicone rubber adhesive of 0.02 inches (0.51 mm)
thickness.

These layers are all bonded together, with layers 51
and 53 and element 52 constituting the heater 24. The
metal foil is in a form similar to a printed circuit
so that it provides a tortuous path for electrical current
which enters and leaves the heating element by terminals
56 as indicated in Fig. 1. The heating element is
similar to elements commercially available under the
trade mark "Thermofoil" as supplied by Minco Products,
Inc., of Minneapolis and used for example in photocopier
machines. The thinness of the heating element and of
the insulating layers associated therewith are such
that this is readily flexible and is capable of being
deformed by the compressive forces applied by the insulating foam rubber layer to conform to the external shape
of the walls 34 of the lens holder, to ensure good
thermal contact between these surfaces and the heater.
The nature of the heater and pad 54 are such that pressure applied at a discrete point on the heater can cause
this to be deformed into the base by an amount at least
equivalent to the heater thickness. The lugs 19 cooperate
with surfaces 30a of the lens holder to ensure that when
in position the base 54 is compressed by an amount equivalent to at least 25% of its thickness.

In the bottom wall 22a of the recess, in the underside,
there is provided a small socket to accommodate a
thermostat 60. This is of the bimetal detector type such as are sold under the trade mark "Microtherm" available from Cantherm of Montreal, Canada. The top of the thermostat is separated by a thin layer of the plastic wall, of less than 0.05 inches (1.25 mm) thickness, from the interior of the recess. This arrangement is provided so that the temperature of the thermostat will respond well to the heat within the recess and which is received by the lens holder so that the thermostat can be calibrated to switch at a consistent lens temperature.

The thermostat 60, and the terminals of the heating elements, as well as the push button 14 and indicating light 16 are connected into a circuit which includes components mounted on a circuit board 62, and which also includes two rechargeable batteries mounted in the main body 12a of the casing but not shown. The circuit also includes a jack shown at 63 in Fig. 1 for connection to a transformer unit itself connected to mains to allow the recharging of the batteries.

Another item in the circuit is a safety switch 65 which is mounted in the surface 20 of the rectangular recess, between the two conical recesses 22. This switch is closed when pressed down by the lens holder plate 30 being inserted within the rectangular recess, and prevents operation of the heaters when the lens holder is not in place.

A suitable circuit for connecting the various electrical parts will now be described with reference to Fig. 5. This circuit includes:-

The batteries B1

Push button switches 14 and 65

A relay RL1

A thermostat 60

The two heating elements shown as 52 and 52'
in Fig. 5

The light emitting diode 16, and
A resistor R1.

The circuit also includes a sub-circuit for recharging the batteries B1, and connected to jack 63, but which is not shown since it is conventional.

The manner in which the circuit operates is as follows:- The lens carrying case 30 is inserted into the recess 18 in the disinfecting unit, which closes the safety switch 65. To start the disinfecting process, the start switch 14 is momentarily depressed. This completes the circuit to the coil of relay RL1 through the normally closed thermostat 60, and the relay switch closes applying current to the heater elements 52 and 52' and also supplying the indicator light 16 which shows that the heaters are energised. The switch 14 can then be released. The resistor R1 limits current through the light emitting diode 16, and these components and relay RL1 are chosen to require very little energy so that most of the energy is taken by the heating elements.

The thermostat 60 is calibrated to open at a temperature which corresponds to a suitable disinfection temperature for the lenses which is about 80°C. When the thermostat opens this breaks the current flow through the coil in the relay so that this returns to the open position so that the heaters are no longer energised and the light 16 goes out. The lens holder however is not removed from the heating unit until this has cooled down sufficiently to be handled, which occurs within several minutes. When the temperature in the vicinity of the thermostat 60 has cooled sufficiently this returns to the normally closed position ready for a new cycle. The whole cycle will take about 15 to 20 minutes, including cooling, which is considerably less than with prior art devices.

Fig. 6 shows a circuit with some additional features which may be used if desired. Specifically, this circuit includes a further thermostat TH2, normally open, in circuit with a second contact of relay RL1, and a diode D4 which is in series with the light emitting diode 16 and resistor R1. The circuit initially operates as described for Fig. 5. However after the thermostat 60 has opened, allowing the relay to move back to the initial position, the second contact of the relay and the thermostat TH2 (now closed since the lens holder is at temperature) maintains the connection between the battery and the light emitting diode 16, with the diode D4 preventing flow of current to the thermostat TH2 through the heaters L1, L2. The thermostat TH2 is calibrated to open when the temperature of the holder has dropped sufficiently for this to be safely handled, at which point the light emitting diode 16 goes out to indicate that the cycle is completed. The second thermostat can be positioned within the second of the recesses in manner similar to that shown for thermostat 60 in Fig. 3, only one thermostat 60 being required for controlling the circuit.

The provision of batteries for the unit is not an essential feature and a small, compact and light unit can be made without batteries.

- 1 -

Claims:

1.    Apparatus for disinfecting contact lenses, comprising in combination:
    (a)  a lens holder including a hollow receptacle and means for locating a contact lens close to the inner surface of said receptacle, and
    (b)  a heat disinfecting unit having a casing with a recess shaped to receive the outer surface of said hollow receptacle, said recess including an electrical heater arranged to contact a substantial part of the outer surface of said receptacle for transmitting heat thereto and thus to disinfect the lens held by the holder,
    (c)  said heater including an electrical conductor thermally isolated from any substantial metal body and separated by thermal insulation from the casing.

2.    Apparatus according to claim 1 wherein said lens holder includes a plate having two of said hollow receptacles protuding from the underside and having means for locating a lens in each receptacle so that the lenses are positioned close to the inner surfaces of said receptacles, said heat disinfecting unit having two recesses shaped and positioned each to receive one of said protuding receptacles, and wherein retaining means are provided for releasably retaining the lens holder in position with the receptacles in pressure engagement with the heaters of the recesses.

3.    Apparatus according to claim 2 wherein said retaining means include surfaces which diverge downwardly and between which said plate is an interference fit, and wherein means are provided giving access to an edge of said plate to allow ready removal by hand.

4.      Apparatus according to claim 2, including a switch normally isolating the heaters, and positioned so as to be closed by said lens holder plate when said plate is positioned in said locating means.

5.      Apparatus according to claim 1 or 2 wherein said means for locating the lens in said receptacle includes a resilient lens support having a convex surface suitable for holding a contact lens, said support being held in place by a removable cap of the receptacle, and wherein the volume of said support is a large part of the total internal volume of said receptacle.

6.      Apparatus according to claim 1, wherein said electrical heater is a flexible element mounted on a resilient thermally insulating base, the flexibility of the heating element in relation to the resilience of the insulating base being such that the heater tends to conform to the shape of the exterior of the lens holder when the latter is held in place in the recess.

7.      Apparatus according to claim 1 or 6 further including means for retaining the lens holder in pressure contact with the heater.

8.      Apparatus for disinfecting contact lenses including a casing having means for locating a contact lens holder, an electrical resistance heater mounted in such position as to contact a lens holder when held by said locating means, the casing having a circuit for connecting the heater to a source of electrical current for energizing the heater and disinfecting a lens held by the holder, in which the heater is a flexible element mounted on a resilient heat insulating base in such manner as to be thermally isolated from any substantial metal body, and wherein said locating means includes means for retaining the holder in pressure contact with the heater in such manner as to compress said base, whereby the heater tends to conform to the shape of the lens holder when the latter is held in place thereon.

9. Apparatus according to any of claims 6, 7 or 8 wherein said base has thickness considerably greater than that of the heater and the heater flexibility is such that the heater can be deformed from the relaxed state by an amount at least equivalent to its thickness by pressure applied to it at a discrete point.

10. Apparatus according to any of claims 6 to 9, wherein the resilient insulating base is formed of sponge rubber.

11. Apparatus according to any of claims 6 to 10, wherein the heater includes a flat heating element of metal foil covered with a layer of electrically insulating material.

12. Apparatus according to any of claims 6 to 8 wherein said heater includes a flat element of metal foil having an insulating layer of synthetic rubber on its two faces, one of said layers being bonded to a layer of sponge rubber providing said insulating base.

13. Apparatus according to any of claims 6 to 12, wherein said heater includes a metal element less than 0.002 inches (0.051 mm) in thickness.

- 4 -

14.    Apparatus according to any of claims 6 to 12,
wherein said heating element includes a flat element of
metal foil of about 0.001 inches (0.025 mm) thickness.

15.    Apparatus according to any of claims 6 to 12,
wherein the total thickness of said heating element is
less than 0.05 inches (1.25 mm), said thickness being substantially less than the thickness of said insulating base.

16.    Apparatus according to any one of claims 8 to 15
wherein said means for locating the lens holder includes
a recess, said heater being mounted in the recess. ·

17.    Apparatus according to claim 1 or claim 16 wherein
said recess is circular and formed within a wall of the
casing which wall is formed of plastic material, said
heater being of annular form surrounding a bottom of the
recess, and wherein a thermostat is mounted on the underside of said wall forming the bottom of the recess so as
to be separated from the interior of the recess by a layer
of said plastic material.

18.    Apparatus according to claim 17 wherein said
thermostat is positioned in a socket in said wall forming
the recess bottom.

19.    Apparatus according to claim 18 wherein the thickness of plastic material in said wall separating the
thermostat from the recess is less than 0.05 inches
(1.25 mm).

20.    Apparatus according to claim 1 or claim 16 wherein
the recess is frusto-conical in form, said heater being
an annular element located on the curved wall of the
recess.

21.    Apparatus according to any of claims 1 to 8 wherein said casing includes at least one cavity for a battery or batteries connectable to energize said heater, said heater or heaters being capable of providing heat to disinfect lenses through several cycles when powered solely by said batteries.

22.    A holder for contact lenses including a plate having two hollow receptacles protuding from the underside thereof, the upper ends of said receptacles having removable caps, each cap carrying a lens support of convex form, said plate having horizontally extending edge portions for engaging with retaining means of a disinfecting unit, and wherein the bottom surfaces of said receptacles are flat to allow the holder to rest in stable manner on a flat surface.

23.    A holder according to claim 22 wherein the volumetric capacity of each of said receptacles for liquid, when said receptacles have a contact lens therein, is less than about one cubic centimeter.

24.    A holder according to claim 22 wherein each said lens support occupies at least 30% of the volumetric space within each said receptacle.

25.    A holder according to claim 22 wherein, when a lens is held by said member within the receptacle, its outer surface is spaced by no more than 0.1 inch (2.5 mm) from the inner surface of the receptacle.

26.    Apparatus according to claim 5 wherein said receptacles have removable caps and wherein the bottom surfaces of said receptacles are flat to allow the holder to rest in stable manner on a flat surface.

FIG. 1

0021828

2/3

FIG.2

FIG. 3

FIG.4

FIG.5

FIG.6